# EUROPEAN PATENT APPLICATION

(11) **EP 1 352 891 A1**
(43) Date of publication of application: **15.10.2003**
(21) Application number: 02447066.8
(22) Date of filing: 12.04.2002
(51) Int. Cl.: C07C 51/09

(54) **A method for the direct hydrolysis of fatty acid esters to the corresponding fatty acids**

(71) Applicant: OLEON, 9940 Ertvelde (BE)
(72) Inventor: Packet, Dirk, 1730 Asse (BE)
(74) Representative: Luys, Marie-José A.H.

(57) **Abstract**

This invention relates to a liquid phase process for hydrolysing a fatty acid ester to its corresponding fatty acid, the method comprising the steps of contacting the fatty acid ester in the liquid phase with water in the presence of an acid catalyst. As a catalyst use is made of a solid acid catalyst selected from the group of zeolites, ion exchange resins and acid amorphous catalysts.

## Description

The present invention relates to a liquid phase process for hydrolysing fatty acid esters to the corresponding fatty acids as described in the preamble of the first claim.

From US-A-5.440.061 a process is known in which a C1-C4 alkyl ester, preferably a methyl ester of a C4-C19 fatty acid is mixed with water, an amount of an acid catalyst and DMSO, to form a single phase reaction mixture. DMSO is added to improve the solubility of water in the fatty phase. Care is taken that the initial molar ratio of water to ester is at least 1:1, and the initial molar ratio of any residual carboxylic acid to ester is less than 1:1. As a catalyst use is made of a highly acidic compound, in particular a sulfonic acid catalyst or sulfuric acid. Hydrolysis takes place by heating the single-phase reaction mixture to a temperature of between about 70 and 130°C.

The process disclosed in US-A-5.440.061 however has the disadvantage that the isolation of the fatty acid from the reaction mixture containing the acid catalyst and DMSO is complicated. According to US-A-5.440.061 first the unreacted water, traces of the volatile alcohol, DMSO and any unreacted ester have to be removed through vacuum distillation. The fatty acid product is then recovered by distillation of under vacuum. The acid catalyst is left behind in the reaction vessel and can be recycled to the reactor.

There is thus a need to a process with which isolation of the fatty acid from the reaction mixture may be simplified.

The object of the present invention is therefore to provide a process for the hydrolysis of fatty acid esters to the corresponding fatty acids, with which the fatty acid may be recovered in a simple manner.

This is achieved according to the present invention with the technical features of the characterising part of the first claim.

In the method of the present invention the hydrolysis of the fatty acid ester to the corresponding fatty acid is carried out in the presence of a solid acid catalyst, in particular a catalyst selected from the group of zeolites, ion exchange resins and acid amorphous catalysts.

The inventor has found that when contacting a fatty acid ester with a solid acid catalyst, surprisingly a hydrolysis of the ester towards the corresponding fatty acid takes place. The inventor has also observed that the conversion of the fatty acid ester towards the fatty acid is reasonably good, takes place within a reasonable period of time and at may be carried out within an acceptable temperature range at which decomposition of the fatty acid ester and/or the fatty acid is negligible.

The inventor is of the opinion that the unexpected good conversion of fatty acid ester to fatty acid can be explained as follows. The acidic sites present on the catalyst surface function as water adsorption sites. The binding of the water to the sites appears to be sufficiently strong to provide adsorption of water to the acidic sites and sufficiently weak to promote the nucleophilic nature of the water molecule and have it available for the hydrolysis reaction. When contacting the fatty acid ester with the catalyst, the fatty acid ester is adsorbed to the catalyst surface. The fatty acid ester - water contact is facilitated by the presence of the adsorbed water on the catalyst surface, thus promoting hydrolysis of the fatty acid ester to its corresponding fatty acid. This in turn entails the advantage that the amount of water needed to obtain good conversion may be significantly reduced as compared to the state of the art process.

Adsorbed water consumed in the hydrolysis reaction can easily be replenished by re-hydrating the catalyst, for example through contacting the catalyst with water or steam. As the contact of the fatty acid ester with the water needed for the hydrolysis reaction takes place on the catalyst surface, the problem of the solubility of water in the fatty acid ester phase has become irrelevant and does no longer adversely effect the reaction rate or conversion of the fatty acid ester to its fatty acid.

The use of a solid acid catalyst entails the advantage of providing a non-corrosive process.

The inventor has furthermore found that the hydrolysis reaction of the present invention may be carried out at moderate temperatures so that decomposition of the fatty acid ester, the fatty acid and the catalyst become negligible.

Solid acid catalysts suitable for use in the method of this invention include zeolites, in particular zeolites having a high SiO₂/Al₂O₃ ratio, which is preferably above 5, more preferably above 10 or 20. The higher the SiO₂/Al₂O₃ ratio, the higher the stability of the zeolite is under hydrothermal conditions. It is however important to find the optimum compromise between the number of acid sites present on the catalyst surface and the acidity of the individual sites, to provide an optimum degree of conversion at a reasonable reaction rate. Zeolite catalysts are particularly preferred because of their good heat stability, which permits the use of higher reaction temperatures of 200°C and more and is determined by the stoichiometric SiO₂ / Al₂O₃ ratio.

Other suitable catalysts include porous ion exchange resins such as for example acid polymeric resins like Amberlyst 15 or Amberlyst 36 (Rohm & Haas), and amorphous acid catalysts such as for example silica, alumina, silica-alumina, silica-zirconia, silica-titania or aluminium phosphate gels.

When selecting an appropriate catalyst for use with the present invention, care should be taken that the amount of extraframework Al species present in the catalyst is sufficiently low. With extra framework Al species there is a risk to de-activation of the catalyst because of the risk to saponification of the fatty acid ester by the extra framework Al species, which blocks the catalyst.

The hydrolysis reaction of this invention is preferably carried out at a temperature of between 200-350°C, preferably between 230-300°C, more preferably between 250-280°C. Below 200°C the reaction rate gets insufficient and is hardly economically interesting. Above 300 or 350°C there is an increasing risk to cracking of the fatty acid ester and fatty acid, as well as to entraining of the fatty acid ester from the reaction mixture with the alcohol and water. In the preferred temperature ranges an optimum balance is provided between process energetics on the one hand and conversion degree and suppressing of unwanted side reactions on the other hand.

The hydrolysis reaction of this invention is preferably carried out at an over-pressure, preferably a pressure between 2 and 100 bar, more preferably between 10 and 70 bar. Below 10 bar, the risk to evaporation of the fatty acid ester and the fatty acid together with the alcohol and water increases, depending on the volatility of the ester. The man skilled in the art will be capable of selecting the adequate pressure at which the fatty acid ester remains in the liquid phase.

Fatty acid esters suitable for use in the process of this invention are esters of:
(I) at least one mono- or polyfunctional alcohol
   and
(II) at least one organic carboxylic acid comprising at least one carboxylic acid group.

The mono- or polyfunctional alcohol may be any of these alcohols known to the man skilled in the art. Examples of suitable mono-functional alcohols include C₁-C₁₀ alcohols, preferably C₁-C₄ alcohols as these have the lower boiling points and escape from the reaction mixture at the usual reaction temperatures. Particularly preferred are the fatty acid methyl esters as the methanol formed in the course of the hydrolysis reaction has a low boiling point and can be easily entrained from the reactor. Examples of suitable polyfunctional alcohols include di- and tri-alcohols, for example glycerol as these are present in the naturally occurring oils and fats, although other polyfunctional alcohols known to the man skilled in the art may also be used.

The organic carboxylic acid moiety of the fatty acid ester comprises at least one COOH group and a hydrocarbon chain having 1-54 carbon atoms, the hydrocarbon chain being aliphatic or aromatic, saturated or unsaturated, branched, straight chain or cyclic, including polycyclic, and containing one or more functional groups. Other suitable carboxylic acids include synthetic mono-, di- or polycarboxylic acids and dimers or polymers of the above-described carboxylic acids.

Preferably, the hydrocarbon chain of the carboxylic acid moiety of the fatty acid ester comprises 6 to 24 carbon atoms. More preferred are those moieties containing 8 to 10 or 16 to 18 carbon atoms.

Examples of suitable methyl esters include C₈-C₁₀ methyl esters and C₁₆-C₁₈ methylesters. The methyl ester reactant herein can be a specific methyl ester or a mixture of different methyl esters. The methyl ester used in the process of this invention is for example a mixture obtained through methanolysis of a naturally occurring fat or oil mixture.

The fatty acid ester reactant used in the process of the present invention can be a specific fatty acid ester or a mixture of different esters, containing different organic carboxylic acids and/or different alcohols.

The amount of catalyst present in the reaction mixture is not critical to the invention, but is preferably at least 5 wt. % with respect to the weight of the reaction mixture in case of a batch process. When operating in a continuous flow process with a fixed bed catalyst, the amount of catalyst will be adapted such that the process may be operated at the desired LHSV.

The catalyst, in particular when used as a fixed bed catalyst, may be used in the form of an extrudate, spheres, tablets or any other form the mechanical strength of which is satisfactory for use with the process of this invention. Particularly useful are monolithic catalysts the surface of which is coated with one or more zeolites. Monolithic catalysts allow a better control of the reaction hydrodynamics in a continuous flow reactor as compared to a randomly packed catalyst bed.

To ensure a sufficiently high conversion rate and conversion degree of the fatty acid ester, care needs to be taken that a sufficient amount of water is adsorbed to the catalyst surface. This may for example be achieved by having steam flown through the reaction mixture. The steam flow should however be adapted such that it the stability of the catalyst is not adversely affected.

To facilitate isolation of the fatty acid fraction from the reaction mixture, preferably use is made of a fixed bed catalyst. In that case the fatty acid fraction may be recovered at high purity through a simple distillation step while simultaneously dispensing with the need to an additional purification step.

In particular when a hydrolysis of fatty acid methyl esters is carried out, the fatty acids can easily be isolated from any unreacted methyl esters through distillation, since the esters have a significantly lower boiling point than the corresponding fatty acids.

The process of the present invention can be carried out as a continuous process or a batch process, but is preferably carried out as a continuous process.

The invention is further elucidated in the following figure and figure description and in the examples given below.

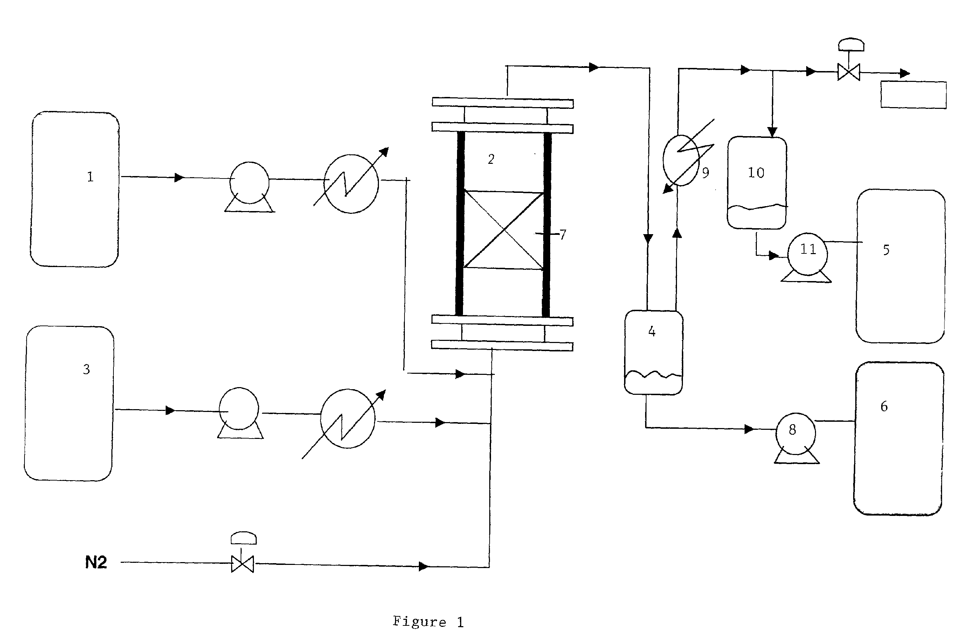

The process can be carried out as is shown in the preferred embodiment of figure 1.

In a possible method for carrying out the process of this invention, an amount of a fatty acid ester 1 in the liquid phase is supplied to a lower part of a reactor 2 containing a fixed bed catalyst 7. The reaction mixture is heated to a temperature of about 240°C and 300°C. Steam 3 is supplied through the reactor bottom. The pressure in the reactor is maintained approximately at 10 bar to keep the fatty acid ester and fatty acid in the liquid phase. The fatty acid ester is hydrolysed to the corresponding fatty acid and alcohol. Because of its lower boiling point, the alcohol evaporates and is entrained from the reactor together with the steam. The alcohol/fatty acid mixture is captured in a second separation vessel 4. The alcohol and steam are evaporated from the mixture. After having been condensed in a condensor 9, the methanol/water mixture is captrued in a container 10, transferred to a cooler 11 and stored in a storage container 5. The fatty acids remain in the bottom and are transferred to a storage container 6 after having been cooled in a cooler 8.

By using a heat stable fixed bed catalyst, the fatty acid fraction will be essentially free of impurities originating from the catalyst. Therefore, a simple distillation may be sufficient in order to obtain a substantially pure fatty acid fraction. Any unreacted fatty acid ester, in particular methyl ester, present in the fatty acid fraction may be separated therefrom by distillation, since the boiling point of the fatty acid ester is substantially lower than that of the corresponding fatty acid differs.

The invention is further elucidated in the following examples.

The Examples describe experiments in which different catalysts were tested for their performance with respect to the hydrolysis reaction of fatty acid esters to the corresponding fatty acids. The conversion of the fatty acid ester to the corresponding fatty acid was measured as a function of time.

### Example 1-5

In Examples 1-5, a batch reactor was loaded with 10 parts by weight of a USY catalyst, obtained from UOP. The catalyst has the following composition: SiO₂ 72.2 wt. %, Al₂O₃ 22.87 wt. %, Na₂O 0.2 wt. %, (NH₄)₂O 4.0 wt. %. The catalyst has a surface area of 770 m²/g.

Before use, the catalyst has been calcined for a period of 12 hours at 550°C to bring is in the acidic form.

The amount of water supplied to the reactor was 5 parts by weight. The reactor contained 85 parts by weight of the methyl ester with the following composition: C6 4.1 wt. %, C8 59.2 wt. %, C9 0.2 wt. %, C10 32.5 wt. %, C12 2.8 wt. %, total methyl ester: 98.8 wt. %.

The reaction mixture was heated to a temperature of respectively approximately 250 °C, 260°C, 270°C, 280°C and 300°C at a pressure of approximately 70 bar in a closed reactor. After a reaction time of 360 min, conversions of respectively 53.6%, 62.7%, 67.3%, 70.7%, and 72.4% of the methyl ester to the corresponding fatty acid were obtained.

### Example 6.

The reaction described in examples 1-5 was carried out in a continuous flow reactor with a continuous steam flow through the liquid phase containing the methyl ester, without adding water at the beginning of the process, at a temperature of 280°C. A conversion of 87.3 % could be obtained.

This improved conversion is explained by the inventor because the alcohol formed in the course of the hydrolysis reaction is continuously withdrawn from the reactor, as a consequence of which the reaction equilibrium is shifted to the right.

### Example 7-9.

Examples 7-9 were carried out similarly to examples 1-5, except that as a catalyst use was made of mordenite zeolites of the type Zeocros CF815A, CF815B obtained from Zeolyst and ZM510 obtained from Zéocat. The properties of these catalysts are listed in table 1 below.

| | CF815A | CF815B | ZM510 | CBV20A |
|---|---|---|---|---|
| SiO₂ wt. % | 83.78 | 90.61 | 92.1 | 95.33 |
| Al₂O₃ wt. % | 15.39 | 7.24 | 7.8 | 4.65 |
| Na₂O wt. % | 0.07 | 0.05 | 0.04 | 0.02 |
| Surface area (m²/g) | 806 | 894 | 530 | 558 |

The reaction mixture was heated in a closed batch reactor to a temperature of approximately 280°C at a pressure of about 70 bar. After a reaction time of 360 min, conversion degrees of respectively 64.6 %, 67.3 % and 90.3 % of the C8/C10 methylester to the corresponding C8/C10 fatty acid were obtained.

### Examples 10-11.

Examples 10-11 were carried out in a closed batch reactor similarly to examples 1-5, except that as a catalyst use was made of an acid resin catalyst type Amberlyst 15 and Amberlyst 36 purchased from Rohm & Haas.

The reaction mixture was heated to a temperature of approximately 120°C, at a pressure of about 10 bar. After a reaction time of 360 min, a conversion degree of the C8/C10 methylester to the corresponding C8/C10 fatty acid of about 29.5 % was obtained, for both catalysts.

The lower conversion obtained with the acid resin catalysts is attributed to the lower reaction temperature used because of the lower thermal stability of the polymeric resin catalyst.

### Example 12.

Example 12 was carried out in a closed batch reactor, similar to examples 1-5, except that as a catalyst use was made of an acid amorphous silica-alumina catalyst J639 from Grace. The catalyst had a surface area of 390 m²/g and contained 1.5 wt. % of alumina.

The reaction mixture was heated in a closed batch reactor to a temperature of approximately 280°C, at a pressure of about 70 bar. After a reaction time of 360 min, a conversion degree of the ester to the corresponding fatty acid of about 31.7 % was obtained.

### Example 13.

Example 13 was carried out similarly to example 6, in a continuous flow reactor with a continuous steam flow through the liquid phase containing the methyl ester, at a temperature of 280°C, except that a mordenite CBV20A type zeolite obtained from Zeolyst was used as a catalyst. A conversion of 88.9 % could be obtained. The properties of the catalyst are listed in table 1 given above.

### Example 14.

Example 14 was carried out similarly to example 6, in a continuous flow reactor with a continuous steam flow through the liquid phase containing the methyl ester, at a temperature of 280°C, a pressure of 10 bar and a LHSV of 1h⁻¹. As a catalyst, use was made of a fixed bed of mordenite CBV20A extrudates with a diameter of 1/16. A conversion of 90.5 % could be obtained.

## Claims

1. A liquid phase process for hydrolysing a fatty acid ester to its corresponding fatty acid, the method comprising the steps of contacting the fatty acid ester in the liquid phase with water in the presence of an acid catalyst, **characterised in that** as a catalyst use is made of a solid acid catalyst selected from the group of zeolites, ion exchange resins and acid amorphous catalysts.

2. A process as claimed in claim 1, **characterised in that** the fatty acid ester is an ester of at least one mono- or polyfunctional alcohol with at least one carboxylic acid comprising at least one carboxylic acid group.

3. A process as claimed in any one of claims 1-2, **characterized in that** the fatty acid ester is an ester of a carboxylic acid having a hydrocarbon chain with 1-54 carbon atoms, the hydrocarbon being aliphatic or aromatic, saturated or unsaturated, branched, linear, cyclic or polycyclic, and containing one or more heteroatoms and/or functional groups.

4. A process as claimed in any one of claims 1-3, **characterized in that** the fatty acid ester is an ester of a carboxylic acid having a hydrocarbon chain with 6 to 24 carbon atoms, preferably 8 to 10 or 16 to 18 carbon atoms

5. A process as claimed in any one of claims 1-4, **characterized in that** the fatty acid ester is an ester of a carboxylic acid, which is a polymer of two or more organic carboxylic acids.

6. A process as claimed in any one of claims 2-5, **characterised in that** the alcohol is a condensation product of two or more alcohol monomers.

7. A process as claimed in any one of claims 1-6 **characterized in that** the fatty acid ester is a methyl ester of the fatty acid.

8. A process as claimed in any one of claims 1-7, **characterized in that** hydrolysis is carried out at a temperature of between 200-350°C, preferably 230-300°C, more preferably 250-280°C.

9. A process as claimed in any one of claims 1-8, **characterized in that** hydrolysis is carried out at a pressure of between 2-100 bar, preferably 10-70 bar.

10. A process as claimed in any one of claims 1-9, **characterized in that** the molar ratio of the amount of water with respect to the amount of fatty acid ester is at least 1.

11. A process as claimed in any one of claims 1-10, **characterized in that** the process is carried out as a continuous process or a batch process.
